# EUROPEAN PATENT APPLICATION

(11) **EP 2 248 552 A1**
(43) Date of publication of application: **10.11.2010**
(21) Application number: 09159581.9
(22) Date of filing: 06.05.2009
(51) Int. Cl.: A61N 5/10

(54) **Method and apparatus for image guided radiotherapy**

(71) Applicant: DKFZ Deutsches Krebsforschungszentrum, 69120 Heidelberg (DE)
(72) Inventor: König, Thomas, 69123 Heidelberg (DE); Oelfke, Uwe, 74909 Meckesheim (DE)
(74) Representative: Altmann, Andreas

(57) **Abstract**

The invention comprises a method and an apparatus for image guided radiotherapy, comprising: an electron beam source as a common source for generating both, an imaging photon beam as well as a therapy photon beam with a photon energy higher than the photon energy of the imaging photon beam. A therapy target and an imaging target are used, the imaging target comprising at least one crystal. The therapy photon beam is generated by irradiating the therapy target with the electron beam source. The imaging photon beam is generated by arranging the imaging target relative to the electron beam source with an angle between a crystal axis of the crystal's lattice and a direction of propagation of the electron beam source. Imaging photon beam is generated through the emission of coherent bremsstrahlung.

## Description

### Field of the Invention

The invention relates to image guided radio therapy (IGRT). In particular, the invention relates to IGRT with combined imaging and radio therapy based on linear electron accelerators (LINAC).

### Prior Art

It is known to use x-ray imaging for medical diagnostic methods. For medical imaging, x-rays are used, which are radiated onto an object, i.e. a patient, wherein an image is established based on the detection of the radiation propagated to the object. X-rays are produced by irradiating a target, for example tungsten or copper, with a beam of accelerated electrons. The electron beam is decelerated by the target and the loss of energy is irradiated in form ofbremsstrahlung. For imaging, comparatively low energies are used for ensuring soft tissue contrast.

As a regulatory, an additional filter for low energy photons is required, which is located downstream the target for minimizing irradiation with very low energy. Such very low energy irradiation is absorbed within the patients body, in particular in superficial regions, and does not propagate through the body for adding any imaging value. Rather, such very low energy irradiation would increase the dose for x-ray imaging without increasing image quality. Thus, a mandatory filter for very low energy radiation in form of a metal plate (e.g. copper or aluminium with a thickness of 2.5 mm) is mandatory for any x-ray device. However, the mandatory filter is not particularly selective and also blocks parts of the spectrum used for imaging purposes, i.e. low energy photons with energies between 10 keV and 100 keV.

For example US 7,197,116 B2 shows an x-ray source for medical x-ray imaging. For therapy purposes, x-ray radiation is used which is provided by similar physical principles. However, in order to allow substantial therapy effects, x-rays with comparatively high energies are used for irradiating a particular object. These high energy x-rays are particularly suitable for cancer therapy. Such high energy x-rays are not suitable for imaging since no precise imaging detection is possible.

Application US 2004/0179648 shows a radiation therapy method in which the radiation beam is trained on a treatment zone for precisely radiating only a particular area of the object. Since no monitoring is possible with such high energy radiation, particular collimator arrangements are used for precisely defining a desired intensity distribution, wherein the therapy region has to be exactly aligned to this distribution. Thus, the therapy beam is directed onto a particular predefined area, to which the object has to be manually aligned to.

Patent US 4,198,570 shows an equivalent arrangement and is focussed on collimators which provide a certain desired dose distribution. In the introduction of this patent, ionization chambers are described for controlling the position of the beam, however, without the possibility to consider the real position of the object. Thus, also US 4,198,570 is focussed on precisely defining the therapy beam, without proposing any further alignment means for exactly positioning the object with regard to the therapy beam.

In application US 2008/0009731 A1, an integrated unit composed of a therapeutic radiation unit and an image recording apparatus is described. The therapeutic radiation unit has a radiation source, which is directed onto an object area. The image recording apparatus comprises another individual radiation source, distinct from the radiation source of the therapy unit. The radiation source of the image recording apparatus is combined with the radiation detector located opposed to the individual radiation unit of the imaging recording apparatus. Thus, US 2008/0009731 A1 proposes two individual devices, one for therapy radiation and one for imaging. Consequently, the distinct devices do not comprise an element, which is common to both devices and functions. The radiation therapy and the imaging are combined by common support for both devices which arranges the imaging device with an offset angle of 90° to the therapy radiation device. Thus, at the same point of time, the imaging device provides a picture of the object area in top view while the therapy device radiates the object perpendicular thereto in a cross sectional projection. Consequently, the parameters of the therapy beam rely on out-of-date imaging information due to the delay between the point of time at which the image has been taken and the point of time at which the therapy device has been moved to the respective imaging projection angle.

As a consequence, the combined radio therapy device of US 2008/0009731 A1 is inherently associated with impositions in particular due to misalignments by movements of the object within the time interval in which the therapy device is moved to the former position of the imaging device. In particular for small therapy areas and for therapy areas exposed to movements like breathing, heart beat or other uncontrollable or controllable movements of a patient, this delay provokes substantial misalignments between the image target area and the area irradiated thereafter.

It is therefore an object of the invention to provide an approach for a combined medical imaging and radio therapy with a higher precision.

### Summary of the Invention

This object is solved by the subject-matter of the independent claims. The concept underlying the invention, according to which the electron beam source as an element most important for the beam direction (and other parameters) is used commonly for both, imaging and radiotherapy. According to the invention, an electron beam source suitable for generating a therapy photon beam is also used for producing an imaging photon beam with a substantially lower photon energy. In order to render an electron beam source for generating a therapy photon beam compatible for imaging purposes, a crystal is used as a target. In this specification the term crystal relates to a single crystal as well as to a quasicrystal. According to the invention, the crystal is arranged to the electron beam source such that the electron beam provided by the electron beam source is emitted in an angle to the crystal axis of the crystals lattice. In this way, the crystal provides coherent bremsstrahlung. In a preferred embodiment, the angle is an acute angle.

By the respective interaction between electron beam and crystal, photons are inherently generated which have an energy suitable for x-ray imaging even though the electron beam source emits an electron beam suitable for generating a therapy photon beam with a high energy by emitting the (high energy) electron beam onto a target commonly used for therapy purposes. In this way, a high energy electron beam for generating a high energy therapy photon beam can also be used for generating a low energy imaging electron beam without substantial adjustments of the electron beam source and the electron beam emit thereby. In other words, the electron beam source for imaging and for therapy are arranged at the same angle and is provided by one common electron beam source for both, an imaging target (target suitable for producing an imaging photon beam) and a therapy target. Using at least one crystal as therapy target allows the use of a high energy electron beam source (for generating the therapy photon beam) for imaging purposes. Since the electron beam source and its electron beam defines all relevant optical parameters like the direction of the therapy photon beam and of the imaging photon beam, the alignment to the imaging/therapy object can be provided with a substantial higher precision as compared to the prior art.

In order to select the actual mode of operation (imaging/therapy), only the target has to be selected and located into the electron beam. Since the cross section of the target is defined by the electron beam near its source (in the range of less than 1 cm) the desired mode (therapy or imaging) can be provided by moving the respective target into the electron beam source wherein the movement is also in the range of the cross section of the targets, that is about 1 cm. In comparison to the prior art, in which the sources (together with the targets) are rotated around a human with an angle of 90°, it is obvious that the invention allows to select a desired mode at the same projection angle within an insignificant time period. Such movements can be provided by an electro mechanical actuator moving a slide, on which both targets are located, wherein the movement is translational. In particular with regard to an embodiment, in which distinct targets are commonly located on a rotating holding device, it is obvious that the movements of the targets which define the delay between imaging and therapy is by far faster than the movements provoking misalignments (breathing etc.). Depending on the speed of the movement, the modes can be changed (i.e. switching from therapy mode to imaging mode or vice versa) with very short delay, e.g. less than 0.5, 0.2, 0.1 seconds or less which reduces misalignments due to a delay to nearly zero or at least to an amount, which is lower than a resolution used in the imaging mode

According to the invention, at least one crystal is used for providing a low energy photon beam for imaging (i.e. the imaging photon beam) based on a high energy electron beam, which is also used for generating a therapy photon beam. According to the invention, the crystal interacts with the electron beam and generates a high amount of coherent bremsstrahlung exhibiting an excess amount of photons in the energy regime suitably for imaging, thus having an average energy which is substantially lower than the energy of the therapy photon beam. The regularity of the crystal is thus responsible for a significant change in the resulting photon spectrum compared to an amorphous or poly-crystalline target as used in the prior art. In single-crystals, the regularity is provided by the periodicity of the crystal. Further, the regularity is provided by the periodicity of sections of the crystal, in particular by a plurality of consecutive sections. Further, crystals comprise lattice regularity within sections of the crystal, in particular in consecutive sections. In particular, the regularity of the crystal is responsible for a significant change in the resulting photon spectrum compared to an amorphous or poly-crystalline target in form of a plate of tungsten or gold as used in the prior art. Therefore, the imaging target is provided as a single-crystal or a quasicrystalline structure. The lattice of such a quasicrystal is regular and may be described by projecting a higher dimensional single-crystal into three-dimensional space. Technically, they represent patterns that fill space without requiring translational symmetry, therefore enabling rotational symmetries other than 2-, 3-, 4-, and 6-fold, that is, in particular, 5-fold symmetries. Thus, a similar effect as with periodic crystal structures can be provided.

According to the invention, an electron beam source is used as a common source for generating both, an imaging photon beam as well as a therapy photon beam with a photon energy higher than the photon energy of the imaging photon beam. A therapy target is used as well as an imaging target, the imaging target comprising at least one crystal. The therapy photon beam is generated by irradiating the therapy target with the electron beam source. The imaging photon beam is generated by arranging the imaging target (comprising the at least one crystal) relative to the electron beam source with an acute angle between a crystal axis of the crystal's lattice and a direction of propagation of the electron beam source. The imaging photon beam is generated as coherent bremsstrahlung resulting from the interaction between the electron beam source's beam and the imaging target.

According to the invention, a method for image guided radiotherapy is provided, comprising the steps: emitting an electron beam onto an imaging target by an electron beam source, the electron beam having an energy suitable for generating a therapy photon beam. The electron beam is emitted onto at least one surface area of at least one crystal providing the imaging target. The electron beam is emitted onto the crystal in an acute angle to a crystal axis of the crystal's lattice. The crystal inherently provides coherent bremsstrahlung upon interaction with the electron beam, the bremsstrahlung providing the imaging photon beam. The imaging photon beam has an energy lower than the energy of the therapy photon beam. In this context, the term "energy" of the imaging/therapy photon beam mainly relates to the energy of the photons of the respective beams and only indirectly relates to the energy provided by power transfer involved with the beam. Further, according to the inventive method, the imaging photon beam is directed towards an object.

According to the invention, an apparatus for image guided radiotherapy is provided, comprising an electron beam source and an imaging target. The electron beam source is oriented towards the imaging target. The electron beam source is adapted to provide an electron beam propagating along a direction towards the imaging target with an energy suitable for generating a therapy photon beam. The imaging target is provided by at least one crystal having at least one surface area oriented towards the electron beam source. The electron beam source and the at least one crystal are arranged to each other, wherein the direction, in which the electron beam source emits the electron beam, is in an acute angle to a crystal axis of the crystal's lattice. The crystal is adapted to provide coherent bremsstrahlung upon interaction with the electron beam as an imaging photon beam. The imaging photon beam exhibits an increased number of photons in the energy range suitable for imaging when compared to the therapy beam, and an object area is located at a side of the imaging target opposed to the electron beam source. Preferably, an amount of the photons of the imaging photon beam have an energy substantially lower than the energy of the therapy photon beam. In an example, the energy of the electron beam is substantially in the range of 3 - 30 MeV and preferentially in the range of 6 - 18 MeV, while the maximum energy of the resulting therapy photon beam generated by an electron beam with the energy range set out above is determined by the maximum electron energy and subsequent filtration of the photon beam according to the energy spectrum desired, and the energy of the imaging photon beam is preferentially in the range of 60 keV to 140 keV, depending on the dimensions and absorption properties of the body region under investigation.

In general, the spectrum of the photon beam emitted onto the object is defined by a mandatory filter for blocking photons with a very low energy. The mandatory filter (e.g. a plate of 2.5 mm aluminium or copper) blocks the section of the spectrum corresponding to the photons with a very low energy (i.e. a unintentional low energy spectrum), which otherwise would only increase the dose for the object and would be absorbed within the object, in particular at the surface region on which the imaging/therapy beam impinges onto the object. The (nearly) complete absorption of the unintentional low energy spectrum (e.g. < 50 keV) within the object results from the low penetration depth of the corresponding photons, wherein the penetration depth decreases with decreasing photon energy. However, since the mandatory filter is not very selective, also a substantial amount of imaging photons is blocked by the mandatory filter, in particular imaging photons of a low energy section of the imaging photon spectrum. The invention allows a compensation of this loss of imaging photons by increasing in particular the section(s) of the imaging photon spectrum, which is (are) unintentionally blocked by the mandatory filter. The inventive use of at least one crystal particularly increases the intensity of the imaging spectrum, which allows to compensate the loss of photons due to the mandatory filter. Further, the increase of photons in low energy sections of the imaging spectrum can be controlled by the selection of particular crystals, by selecting the angle of incidence of the electron beam emitted onto the crystal (i.e. angle between crystal lattice direction and direction of propagation of the electron beam), or by a combination thereof. This allows to increase low energy spectrum sections of the imaging spectrum according to the unintentional resulting from the mandatory filter. Further, the invention allows to focus the generation of imaging photons on low energy sections of the imaging spectrum and, consequently, the relative reduction of undesired spectrum sections below and above the imaging spectrum (with the lower ones to be blocked by the mandatory filter). This focussing on the imaging spectrum, together with the reduction of the unintentional low energy spectrum provided the inventive use of at least one crystal as target, allows to use other mandatory filters, e.g. filters with diminished blocking properties in low energy sections of the imaging spectrum. Since the invention allows to provide a photon spectrum, in which the imaging spectrum is separated from the unintentional spectrum and the unintentional spectrum is particularly reduced while providing a imaging spectrum with high intensity, the mandatory filter can be reduced in its blocking properties. In particular, this allows to use a more transmissive mandatory filter increasing the efficiency in imaging beam generation, however without increasing the intensity of the unintentional low energy spectrum. In general, the invention firstly allows to define the spectrum of the generated imaging photon beam and secondly increases the energy of the imaging photon spectrum in relationship to the unintentional low energy spectrum. This lowers the requirements for blocking properties of the mandatory filter.

According to a preferred aspect, the inventive method comprises: generating the therapy photon beam by emitting the electron beam onto a therapy target. The electron beam is emitted onto the therapy target and onto the imaging target alternatingly, subsequently or concurrently. The therapy target provides the therapy photon beam upon interaction of the therapy target with the electron beam. The embodiment further comprises the step of directing the therapy photon beam towards the object.

According to a similar aspect of the invention, the apparatus further comprises: the therapy target and a target holder. The therapy target as well as the imaging target are affixed to the target holder. The target holder is movably supported relative to the electron beam source and provides at least one translational or rotational degree of freedom with regard to the electron beam source, i.e. with regard to the main direction of propagation of the electron beam source. The apparatus comprises an actuator connected to the target holder as well as an controller connected thereto, the controller being adapted and connected to control the actuator to selectively exchange the imaging target and the therapy target alternatingly, or subsequently, and to accordingly place the respective target into the direction of propagation of the electron beam. In order to emit the electron beam onto the therapy target and onto the imaging target concurrently, the both targets are arranged by the holder within the electron beam concurrently. In this embodiment, both targets are affixed to the holder, wherein the electron beam is provided in a beam cross section and at least one or a plurality of crystals are arranged between or on the therapy target. This can be provided by a plate of tungsten, copper or a similar material suitable as therapy target, on which at least one diamond or other crystal is arranged, or wherein the at least one crystal is affixed within a recess provided in the plate. Alternatively, the holder can comprise a first holder element onto which the at least one crystal is affixed and a second holder element to which the therapy target is attached, wherein the holder and both holder elements arranged the targets on top of each other and within the trajectory of the electron beam. At least the target arranged closer to the electron beam source comprises passages or openings through which the electron beam can propagate without substantial interaction, wherein the target arranged farther away is arranged behind the passages or openings to be directly emitted by the electron beam source.

In an embodiment, the inventive method further comprises the step of arranging the imaging target between the electron beam source and the object, when the imaging photon beam is provided, and arranging the therapy target between the electron beam source and the object, when the therapy photon beam is provided. The electron beam is emitted onto the therapy target and onto the imaging target alternatingly or subsequently. The imaging target is replaced by the therapy target and vice versa by translational or rotational movement of the target holder to which the imaging target and the therapy target are commonly attached. The target holder can be moved by an actuator like a linear actuator or a motor. The actuator receives signals from a control, by which the mode (therapy or imaging) can be selected or controlled.

According to the further aspect of the invention, the electron beam is emitted as a divergent beam. Alternatively or in combination therewith, the electron beam is scanned over a solid angle. The actual electron beam width can be increased by these measures. The electron beam is emitted onto a surface area of one or of a plurality of or an array of crystals. If the imaging target is provide by a plurality of crystals, these are preferably arranged in a plane perpendicular to the electron beam. Thus, the imaging target holder arranges a plurality of crystals within a plane substantially perpendicular to the direction of propagation of the electron beam. The at least one crystal is preferably provided a thin crystal having a thickness of not more than 200 µm, preferably not more than 100 µm, and particularly preferred of not more than 50 µm According to a particular embodiment, the at least one crystal has thickness in the range of 5 - 50 µm

In order to enable the electron beam source to provide an electron beam with a substantial cross sectional area, the electron beam source is adapted to emit the electron beam as a divergent beam. This can be provided by an electron source having a collimator device, which allows a certain degree of divergence, e.g. an angle of divergence of at least 0.25°, 0.5°, 1°, 1.5°, 2° or 3°. Instead of a collimator device, the angle of divergence can be provided by the acceleration field of the electron beam source and the geometry of the electron beam source.

In addition or as an alternative, the inventive apparatus further comprises an electron beam deflector having a magnetic or electrostatic field generator adapted to provide a changing magnetic or electrostatic field deflecting the electron beam emitted by the electron source. This deflector is adapted to scan the electron beam over a solid angle. The at least one of the crystal has a surface area oriented towards the electron beam source. In case of a plurality or an array of crystals, the crystals are arranged in a plane perpendicular to the direction of the electron beam.

The at least one crystal used within the invention as imaging target is of elemental solid matter. In the context of the invention, the phrase "at least one crystal" relates to one single-crystal or a plurality thereof, one quasicrystal or a plurality thereof, and any combination of (a) single-crystals) and (a) quasicrystal(s). The at least one crystal comprises structures atoms arranged in regularity. Because of this non-random alignment in quasicrystals, in contrast to amorphous bodies, a similar effect as with periodic crystal structures can be provided, especially when the electron beam is incident close to such a symmetry axis. In particular, a quasicrystal can be regarded as having a structure comprising symmetries, and, in consequence, provides comparable effects on electron beams (and the bremsstrahlung generated thereby) as single-crystals.. The at least one crystal, onto which the electron beam is emitted, is preferably made of Be, B, Diamond, Graphite, Si, Mg, Se, Ti, Cr, Fe, Ni, Cu, Ge, Z, Yr, Nb, Mo, Pd, Ag, Ho, Ta, W, Au, Tb or Be₂C. If the crystal used within the invention comprises at least one quasicrystal, it is preferably made of aluminium alloys, Al-Li-Cu, Al-Mn-Si, Al-Ni-Co, Al-Pd-Mn, Al-Cu-Fe or Al-Cu-V or is preferably made of Cd-Yb, Ti-Zr-Ni, Zn-Mg-Ho, Zn-Mg-Sc, In-Ag-Yb or Pd-U-Si. In particular, Al-Mn-Si in icosahedral configuration is suited for providing the at least one crystal. The at least one crystal can be provided as a thin film generated by chemical or physical deposition techniques, the film covering a substrate, preferably a substrate allowing to control of the main direction of growth of the crystals, such that the crystals are aligned to each other and mutually provide parallel crystal axes.

According to a further aspect of the invention, the at least one crystal is inclined to the electron beam or the beam part impinging thereon by a predefined angle, for example an acute angle. The inclination as well as the angle between crystal and beam is defined by the orientation of the lattice of the crystal relative to the electron beam impinging thereon. In particular, the inclination and the angle are defined by the spatial orientation of an axis (e.g. a main axis) of the crystal relative to the direction of propagation of the electron beam. The surface of the crystal can differ from an axis defined by the lattice. Further, the ideal angle used within the invention depends on the electron beam's energy. This ideal angle increases with decreasing electron beam energy. According to a first aspect of the invention, the angle in which the electron beam is emitted onto the crystal lattice is an acute angle. This angle defining the geometric orientation between crystal (i.e. crystal lattice or crystal axis) and electron beam source, is at least 0.05°, at least 0.1°, at least 0.15° or at least 0.2°. Preferably, the angle is not greater than 10°, not greater than 5°, not greater than 2° or not greater than 1.8°. According to a second aspect of the invention, the angle is less than 90°, 45°, 30°, 20°, 15°, 10° or 5°. The second aspect of the invention particularly relates to low electron beam energies of less than 10 MeV, 8 MeV, 6 MeV or 5 MeV, and, according to a preferred embodiment, to electron beam energies of 5 - 6 MeV. The at least one surface area of the at least one the crystal extends in a plane perpendicular to one of the crystal axes, e.g. the main crystal axis or another crystal axis. In particular, the angle is defined by the principal direction of the crystal's lattice on which the electron beam is emitted, and the direction of propagation of this electron beam. For diverging electron beams, outer beam sections have a direction of propagation different to the main propagation direction. In order to compensate for this, each crystal has a radiated surface on which the therapy photon beam is emitted onto or has a or a principal direction of the crystal's lattice, wherein the radiated surfaces (and the principal direction) are inclined to a centre line of propagation of the electron beam by a radial angle of inclination increasing with the distance between the particular radiated surface of the radiated surfaces and the centre line of propagation. The increase is preferably suited to arrange the crystal (i.e. the crystal lattice) in a predefined angle to the electron beam impinging thereon, independent from the distance between crystal and centre line. This increase can be an increase proportionally to the distance or can reflect a relationship between distance and angle according to which the angle is a arccos-function of a value reflecting the distance. The inclination between crystal direction (i.e. direction along which a highly periodic structure is given for a single-crystal or direction of a symmetry axis of a quasicrystal) and the electron beam is particularly relevant for the energy distribution of the imaging photon beam. It could be found that the energies of the peaks for the low energy spectrum are approximately proportional to the tilt angle. According to a particular example, this relates to a 6 MeV electron beam and an energy spectrum of 0 - 100 keV. The area as well as the height of peaks within this low energy spectrum for an inclination of 1° - 2° are approximately twice the area / height of peaks for an inclination of 3° - 4° and, more importantly, are located at different energies within the low energy spectrum for different angles. This dependency can be used to precisely define and select the energy distribution of the low energy spectrum or the amount of energy or intensity of the imaging photon beam by appropriately inclining the imaging target with regard to the electron beam. In order to properly incline the imaging target, the inventive apparatus comprises a tilting mechanism (which can be combined with the holder) for tuning the acute tilting angle according to a given, desired low energy spectrum or amount of total intensity of a low energy component of the imaging photon beam in relationship to a high energy component of the imaging photon beam.

According to a further aspect of the invention, the electron beam is directed onto the imaging target, wherein, dependent of the imaging target material, the thickness of the target material and the energy of the electron beam, a residual electron beam remains after the interaction between the at least one crystal and the electron beam. This residual electron beam is directed onto the object, which, however, is not desired. Therefore, the present invention further comprises to deflect any residual electron beam away from the object by applying an electrostatic field or a magnetic field (or a combination thereof) to the residual electron beam. Thus, the area behind the imaging target (as seen from the electron beam source) can be exposed to an E-field and/or a B-field deflecting the residual electron beam away from the object. These deflection fields have no impact on any photon beams (including the imaging photon beam and the therapy photon beam). In addition, the absorption of the residual electron beam results in undesired bremsstrahlung which mainly propagates in the direction of the residual, decelerated electron beam. Thus, the field used for deflection is arranged to deflect the residual electron beam away from the target and onto an absorbing element. Further, an absorbing element comprising an electrode for attracting the electron beam as well as an absorber (downstream the electrode) for absorbing the bremsstrahlung generated within the electrode can be used for blocking (i.e. absorbing) the deflected electron beam and the bremsstrahlung generated by the deceleration (absorption) of the deflected electron beam (in the following: absorption bremsstrahlung). In addition, a deflection radiation absorber element can be provided for blocking (i.e. absorbing) any radiation (in the following: deflection radiation) resulting from the deflection of the residual electron beam, which propagates towards the object. However, since the radiation resulting from deflection (i.e. the deflection radiation) has very low energies (microwave wavelengths, far IR), which are not critical to a patient, the deflection radiation absorber element is not mandatory. The field providing the deflection can be used to displace the point or region from which the deflection radiation is emitted and to direct the deflection radiation away from the object. At the same time, the field is used for directing the residual electron beam away from the patient, to generate the resulting absorption bremsstrahlung in a direction away from the patient and to block the residual electron beam / absorption bremsstrahlung. The electrode and the absorber together forming the absorbing element are displaced from the imaging photon beam and are arranged to absorb the undesired bremsstrahlung, i.e. the absorption bremsstrahlung of the residual electron beam. According to an exemplary embodiment, a magnetic field is used for deflection, which deflects the residual electron beam about an angle of 45° to a first direction ("to the left"). The resulting undesired bremsstrahlung(absorption bremsstrahlung) is emitted in this angle, i.e. in the direction of the deflected residual electron beam. Preferably, the deflection field provides a deflection which results in a reversal of the propagation direction of the residual electron beam. Thus, the deflection field may provide a deflection of about 180° redirecting the residual electron beam towards the electron source, however, with a lateral displacement to the electron beam emitted onto the target.

In general, the term "electron beam" without additional definitions relates to the electron beam emitted by the electron source before being emitted onto the target. The term "residual electron beam" relates to a part of the electron beam remaining after having passed the imaging target. The term "coherent bremsstrahlung" and "imaging photon beam" relates to the bremsstrahlung generated by the electron beam emitted onto the imaging target. The term "therapy photon beam" relates to the bremsstrahlung generated by the electron beam emitted onto the therapy target. The terms "deflected residual electron beam" and "deflected electron beam" relate to the residual electron beam deflected by the deflecting device and the field(s) generated thereby. The term "absorption bremsstrahlung" relates to the bremsstrahlung generated by the deflected electron beam, which impinges onto the absorber element. The term "absorption bremsstrahlung" neither relates to the imaging photon beam nor to the therapy photon beam. The term "deflection radiation" relates to the low energy radiation generated by the deflection of the residual electron beam.

Accordingly, the apparatus according to the invention comprises a deflector device arranged between the object area and the imaging target. The deflector device comprises a electrostatic or magnetic field generator for deflecting any residual electron beam remaining from the electron beam after interaction with the imaging target away from the object area. In particular, the field can be generated by applying a suitable voltage to the electrode of the absorbing element. Further, the deflection device can comprise an E-field generator (e.g. in form of a voltage source connected to the electrode of the absorbing element, together with the electrode itself) and a B-field generator in form of at least one coil generating a magnetic field to the residual electron beam, i.e. to the region downstream the target. The apparatus comprises a blocking element (or an absorbing element) being arranged and adapted to absorb the deflected residual electron beam and the absorption bremsstrahlung generated within the absorbing element. In addition, a deflection radiation absorber element suited for absorbing microwave radiation and/or far infrared radiation can be used. This deflection radiation absorber element is arranged and adapted to absorb the additional bremsstrahlung produced by the interaction between the deflector device and residual electron beam. These absorbing components can be combined. The absorption element can comprise a shielding, e.g. a layer of metal like lead and is suited to absorb the absorption bremsstrahlung generated in an electrode on which the undesired residual electron beam (after having passed the target, e.g. the imaging target) is deflected onto. In the case of a combination of the absorbing element (absorbing the residual electron beam and the corresponding absorption bremsstrahlung) and the deflection radiation absorber element the respective components can be provided by a shielding on form of an aperture through which the imaging/therapy photon beams can propagate, the aperture being arranged between object and deflecting field.

According to another aspect of the invention, the imaging target is cooled by a heat sink. The heat sink can be based on fluidic nitrogen. The resulting cooling effect controls the temperature of the crystals on a desired maximum temperature, e.g. room temperature, 50° C or even approx. 77 K or less, e.g. 4K. The maximum temperature is given by the thermal resistance of the material and structure providing the imaging target, e.g. by a norm temperature up to which the imaging target can be used properly without compromising the integrity of the at least one crystal of the imaging target. Due to the thermal motion of the crystal atoms, the intensity of the coherent part of the radiation decreases with the temperature (depending on material parameters of the crystal, in particular on the Debye-Waller factor of the crystal's atoms). Therefore, it is preferred to actively cool the imaging target using a heat sink suited and arranged for absorbing heat generated within the imaging target. In addition, the imaging target is more temperature sensitive than the therapy target due to its crystal properties. Therefore, the inventive method comprises emitting the electron beam onto the imaging target with an intensity substantially lower than the intensity used for emitting the electron beam onto the therapy target. In this view, the present invention comprises the step of reducing the intensity of the electron beam when the therapy target as emission target for the electron beam is exchanged by the imaging target or before this exchange. Likewise, the intensity of the electron beam is increased, when the imaging target as emission target for the electron beam is exchanged by the therapy target or after this exchange has been completed. Accordingly, the apparatus of the invention comprises a synchronizer for synchronizing the movement of the targets and the intensity of the electron beam source. The synchronizer is connected to both to the electron beam source as well as to the actuator moving the targets (or moving the target holder). The synchronizer is preferably connected to the control controlling the target movement or can be provided together with the control as one control unit.

In case of substantially thermoresistant crystals as imaging targets, the intensity of the electron beam does not have to be reduced. In particular, if thermoresistant crystals are used, the targets do not have to be exchanged and the therapy target is obsolete. Further, an imaging target and a therapy target (if comprising thermoresistant crystals) can be exposed to the electron beam concurrently. Thermoresistant crystals can be provided by crystals having a high thermal conductivity and/or having a sufficient thickness of about 5 mm, 2 mm, 1 mm or 0,5 mm. in order to withstand the mechanical stress resulting from the temperature. At the same time, the thickness of the crystal is preferably at most 5 mm in order to avoid an undesired reduction of imaging photons, i.e. photons with low energies, e.g. less than 100 keV or 80 keV. This undesired reduction increases with the thickness of the imaging target, i.e. the thickness of the at least one crystal.

In addition, a cooling system should be provided, similar to known x-ray devices and radiotherapy devices. Any components of the cooling system are preferably arranged outside the area through which the electron beam and the imaging/therapy beam propagates. According to a particular embodiment, a plate of crystalline metal (single-crystal) like W or a plate of Si (single-crystal), of diamond (e.g. as a matrix of single crystals supported by a heat resistant holder, e.g. of metal, or a single slice of diamond or a similar crystalline or quasicrystalline material with a sufficient thickness (e.g. less than 5 mm or less than 3 mm or less than 2 mm) can be used as imaging target. Preferably, the plate or slice has thickness of less than 1 mm and more than 50 µm, 100 µm or 200 µm Since the spectrum contains both, low energy coherent bremsstrahlung with a high intensity and high energy (incoherent) bremsstrahlung, exposing such a target to the electron beam provides the imaging photon beam (the low energy part of the spectrum) with a high intensity in relation to the intensity of the therapy photon beam (the high energy part of the spectrum). In comparison to the prior art, the invention provides a higher amount of intensity suited for imaging in relation to the amount of intensity not suited for imaging within the imaging beam due to the generation of coherent bremsstrahlung by the at least one crystal.

According to a further aspect of the invention, the imaging photon beam propagating through the object is partly absorbed within the object according to the object's structure and properties and is detected after having passed the object. The detected imaging photon beam (in particular its intensity distribution) provides the imaging data. Thus, the apparatus according to the invention comprises an imaging detector sensitive to the (lower part of the) energy spectrum of the imaging photon beam. The imaging detector comprises an arrangement of detector elements, each element providing intensity, photon number and/or photon energy information. The arrangement provides an image signal at an output of the detector corresponding to the intensity, photon number and/or photon energy distribution detected by the detector elements. An alignment device of the inventive apparatus is connected to the imaging detector and the photon beam source for comparing the image data received by the imaging detector and the current intensity distribution of the electron beam, which is inherently linked with the intensity distribution of the therapy photon beam at the object. In particular, the alignment device provides a comparison (e.g. an overlay image of imaging data and an image of the therapy photon beam's intensity distribution at the object) between imaging data and the intensity distribution of the therapy photon beam at the object.

The invention also relates the following items:
1. Method for image guided radiotherapy, comprising the steps: an electron beam source emitting an electron beam onto an imaging target, the electron beam having an energy suitable for generating a therapy photon beam, characterized by the step of: emitting the electron beam onto at least one surface area of at least one crystal providing the imaging target, wherein the electron beam is emitted onto the crystal in an angle to a crystal axis of the crystal's lattice, the crystal providing coherent bremsstrahlung upon interaction with the electron beam as an imaging photon beam with an energy lower than the energy of the therapy photon beam ; and directing the imaging photon beam towards an object.
2. Method of item 1, further comprising the step of: generating the therapy photon beam by emitting the electron beam onto a therapy target, wherein the electron beam is emitted onto the therapy target and onto the imaging target alternatingly, subsequently or concurrently, and the therapy target provides the therapy photon beam upon interaction of the therapy target with the electron beam ; and the step of directing the therapy photon beam towards the object.
3. Method of item 2, further comprising the step of: arranging the imaging target between the electron beam source and the object, when the imaging photon beam is provided, and arranging the therapy target between the electron beam source and the object, when the therapy photon beam is provided, and wherein the electron beam is emitted onto the therapy target and onto the imaging target alternatingly or subsequently, and the imaging target is replaced by the therapy target and vice versa by translational or rotational movement of a target holder on which the imaging target and the therapy target are arranged.
4. Method of one of the preceding items, wherein the electron beam is emitted as a divergent beam or is scanned over a given solid angle and emitted onto the surface area of one or a plurality or an array of crystals, the plurality of crystals being arranged in a plane substantially perpendicular to the electron beam.
5. Method of one of the preceding items, wherein the angle between the electron beam and the crystal axis is at least 0.05°, at least 0.1°, at least 0.15° or at least 0.2°, and being not greater than 60°, not greater than 50°, not greater than 45°, not greater than 30 °, not greater than 20°, not greater than 15°, greater than 10°, not greater than 5°, not greater than 2° or not greater than 1.8°, and the at least one surface area extends in a plane perpendicular to the crystal axis or to another axis of the crystal, e.g. the main crystal axis, or wherein the angle is an acute angle.
6. Method of one of the preceding items, wherein the electron beam is emitted onto at least one crystal of elemental solid matter or onto at least one quasicrystal providing the crystal, wherein at least one crystal of elemental solid matter providing the imaging target is preferably made of Be, B, Diamond, Graphite, Si, Mg, Se, Ti, Cr, Fe, Ni, Cu, Ge, Z, Yr, Nb, Mo, Pd, Ag, Ho, Ta, W, Au, Tb or Be₂C, and the at least one quasicrystal providing the imaging target is preferably made of aluminium alloys, Al-Li-Cu, Al-Mn-Si, Al-Ni-Co, Al-Pd-Mn, Al-Cu-Fe, Al-Cu-V or of Cd-Yb, Ti-Zr-Ni, Zn-Mg-Ho, Zn-Mg-Sc, In-Ag-Yb, or Pd-U-Si.
7. Method of one of the preceding items, wherein the electron beam is emitted onto the at least one crystal providing the imaging target, wherein the at least one crystal is provided with a thickness along the direction of propagation of the electron beam of not more than 1 mm, 0.5 mm, 200 µm, 100 µm, 50 µm or 20 µm and at least 500 nm, 1 µm, 2 µm, 5 µm or 100 µm or in a range of 5 µm and 50 µm.
8. Method of one of the preceding items, further comprising: directing any residual electron beam remaining after the interaction between the at least one crystal and the electron beam emitted thereon away from the object by applying an electrostatic or magnetic field or a combination thereof to the residual electron beam , by absorbing the residual electron beam in an absorbing element and by absorbing substantially all photons of an absorption bremsstrahlung generated by the step of absorbing the residual electron beam, wherein substantially all photons generated by the application of the field as deflection radiation are blocked or produced to propagate in a direction away from the object.
9. Method of one of the preceding items, further comprising: cooling the at least one crystal by a heat sink, preferably by a heat sink based on heat transfer to a medium, the medium comprising air of the environment, water, oil, fluidic nitrogen, fluidic helium, carbon dioxide.
10. Apparatus for image guided radiotherapy, comprising: an electron beam source and an imaging target, the electron beam source being oriented towards the imaging target **characterized in that** the electron beam source is adapted to provide an electron beam propagating along a direction towards the imaging target with an energy suitable for generating a therapy photon beam, wherein the imaging target is provided by at least one crystal having at least one surface area oriented towards the electron beam source, the electron beam source and the at least one crystal are arranged to each other with the direction, in which the electron beam source emits the electron beam being in an acute angle to a crystal axis of the crystal's lattice, the crystal thereby being adapted to provide coherent bremsstrahlung upon interaction with the electron beam as an imaging photon beam with an energy lower than the energy of the therapy photon beam, wherein an object area is located at a side of the imaging target opposed to the electron beam source.
11. Apparatus of item 10, further comprising: a therapy target and a target holder, the therapy target as well as the imaging target being affixed to the target holder, wherein the target holder is movably supported relative to the electron beam source for providing a translational or rotational degree of freedom with regard to the electron beam source, the apparatus further comprising an actuator connected to the target holder as well as a controller connected thereto, the controller being adapted and connected to control the actuator to selectively exchange the imaging target and the therapy target alternatingly, subsequently or concurrently, and to accordingly place the respective target into the direction of propagation of the electron beam.
12. Apparatus of item 10 or 11, wherein the electron beam source is adapted to emit the electron beam as a divergent beam, or wherein the apparatus further comprises a electron beam deflector having a magnetic or electrostatic field generator adapted to provide a changing magnetic or electrostatic field deflecting the electron beam emitted by the electron source and adapted to scan the electron beam over a given solid angle, the at least crystal being by one, a plurality or an array of crystals, each having a surface area oriented towards the electron beam source, wherein, in case of the plurality or an array of crystals, the crystals are arranged in a plane perpendicular to the direction of the electron beam.
13. Apparatus of one of items 10 - 12, the at least one crystal is of at least one chemical element or is a quasicrystal.
14. Apparatus of one of items 10 - 12, further comprising a deflector device arranged between the object area and the imaging target, the deflector device comprising an electrostatic field generator and/or an magnetic field generator for deflecting any residual electron beam remaining from the electron beam after interaction with the imaging target away from the object area , the apparatus further comprising an absorbing element suitably arranged relative to the deflected electron beam and to the deflector device to receive substantially the complete residual electron beam , the absorbing element comprising an electrode receiving the residual electron beam , and an absorber located behind the electrode arranged relative to the electrode suitable to receive substantially all absorption bremsstrahlung generated by the deflected residual electron beam within the electrode; and a deflection radiation absorber element being arranged and adapted to absorb deflection radiation produced by the interaction between the deflector device and residual electron beam , wherein the deflector device is arranged and adapted to direct the residual electron beam and the deflection radiation away from the object area.
15. Use of an electron beam source as a common source for generating both, an imaging photon beam as well as a therapy photon beam with a photon energy higher than the photon energy of the imaging photon beam by using a therapy target and an imaging target comprising at least one crystal, wherein the therapy photon beam is generated by irradiating the therapy target with the electron beam source and the imaging photon beam is generated by arranging the imaging target relative to the electron beam source with an angle between a crystal axis of the crystal's lattice and a direction of propagation of the electron beam source, thereby generating the imaging photon beam as coherent bremsstrahlung resulting from the interaction between the electron beam source's beam and the imaging target.

### Brief Description of the Drawings

Figures 1a and 1b show the path of rays of a system realized according to the invention for illustrating the inventive method;
Figure 2 shows an embodiment of the apparatus according to the invention in a symbolic representation; and
Figure 3 shows the path of rays for a preferred embodiment of the invention with a diverging electron beam.

### Description of the Drawings

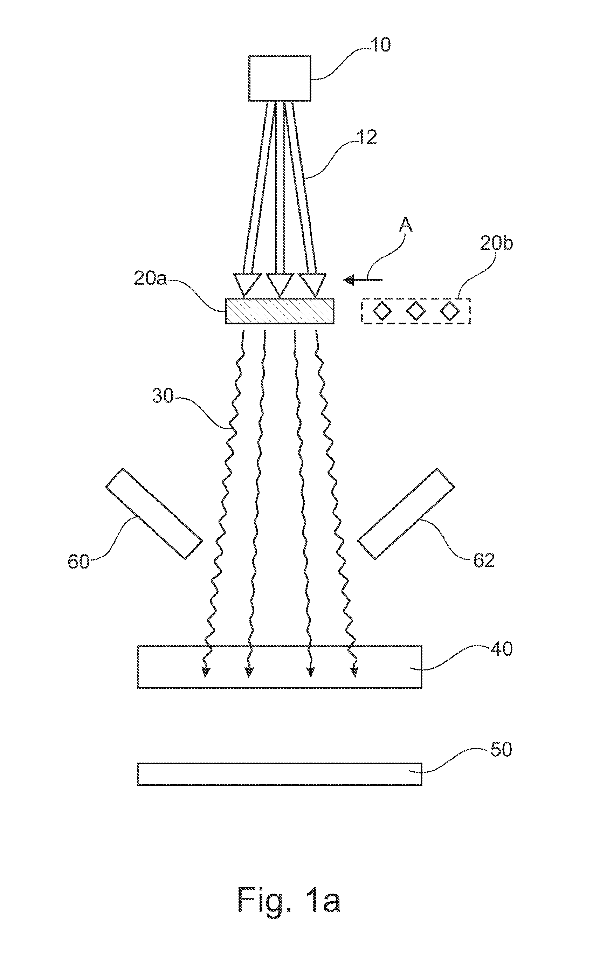

Figure 1a shows the path of rays in a system implemented according to the invention. The system of figure 1a comprises an electron beam source 10 emitting an electron beam 12 onto a therapy target 20a. The electron beam 12 has high intensity illustrated by thick arrows. Further, the electron beam is diverging. The side of the therapy target 20a, which is directed towards the electron beam source, receives substantially all of the accelerated electrons of the electron beam emitted by the electron beam source 10. Irradiating an electron beam 12 onto a thick plate (for example with a thickness of 1 cm) of tungsten or copper by a high energy, high intensity electron beam source (for example a linear electron accelerator, LINAC) 10 is an approach which is also used in the prior art. The electron beam 12 is decelerated by the target 20a. The interaction of electron beam 12 and therapy target 20a provides bremsstrahlung 30 propagating along the same main direction as the electron beam 12. It is to be noted that the electron beam 12 is a beam of charged particles, whereas therapy photon beam 30, equivalent to the bremsstrahlung generated by the target 20a, consists of photons, in particular photons with a high energy for therapy purposes. The therapy beam 30 is radiated onto an object 40, for example an area of a patient's body. In the body, the therapy photon beam 30 is absorbed as depicted in figure 1a. Further, a part (not shown) of the therapy photon beam 30 passes the object 40. However, this part of the therapy photon beam is mostly unsuitable for imaging purposes due to the high energy of the therapy photon beam 30 and the resulting poor soft tissue contrast. Therefore, in the mode shown in figure 1a, a detector plate 50 arranged downstream the object 40 provides sub-optimal images. Further, absorbing elements 60 and 62, 62' are deactivated in the mode shown in figure 1a. The absorbing elements 60 and 62, 62' for absorbing an residual electron beam, the absorption bremsstrahlung generated thereby, and for absorbing deflection radiation are not used in the mode shown in figure 1a since the therapy target 20a (due to its thickness and material properties) does not allow electron beam parts to travel through the complete target 20a. In particular, deflection radiation absorber element 60 is not mandatory for carrying out the invention, independent from the selected mode of operation (therapy or imaging).

According to the invention, the system applied for realizing the invention further comprises an imaging target 20b, which is not located in the path of the electron beam 12 in the mode shown in figure 1a. Thus, the mode shown in figure 1a is suited for generating therapy photons with high intensity and high energy. As can be seen from figure 1a, the therapy target 20a can be replaced by the imaging targets 20b. The replacement can be carried out by moving the targets along direction A.

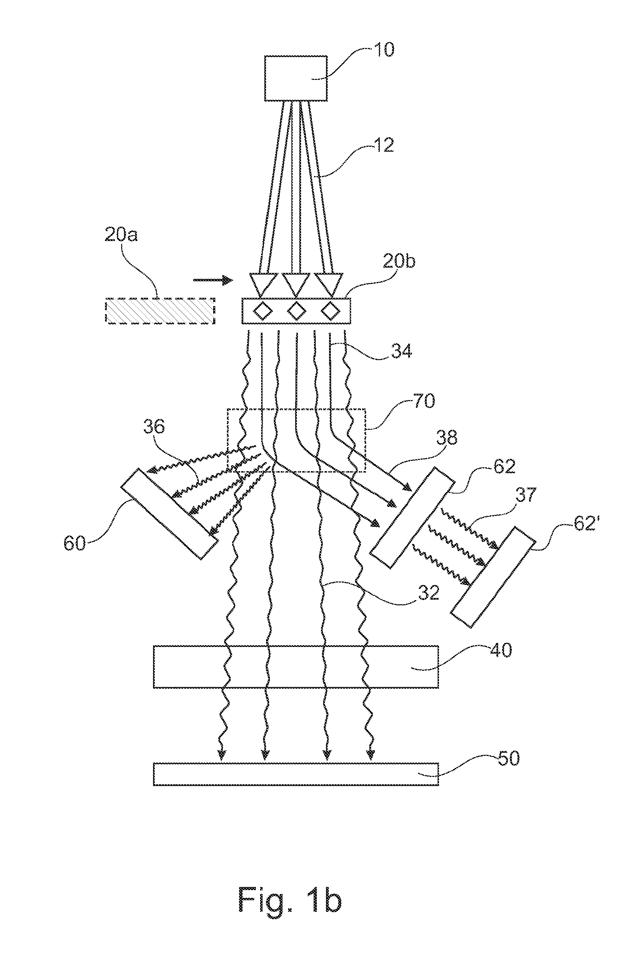

In figure 1b, the same system is shown, however operating in a different mode. Similar to the depiction of figure 1a, figure 1b shows the system with an electron beam source 10, targets 20a (therapy target) and 20b (imaging target), a photon beam 32 provided as bremsstrahlung resulting from the interaction of electron beam 12 and the respective target (here: 20b), the object 40, the imaging detector 50 and the absorbing elements 60 and 62. These system components are similar or identical to the components shown in figure 1a. Therefore, the same reference signs as in figure 1a are used. As to figure 1a, deflection radiation absorber element 60 is not mandatory for carrying out the invention since the deflection radiation is less critical to a patient and is absorbed by thin sheets of metal or by a casing (not shown) of the inventive apparatus.

In particular, the electron source 10 of figures 1a and 1b are identical, however, in different modes of operation. Similar to the depiction of figure 1a, the electron beam source in figure 1b emits an electron beam source with comparably high kinetic energies in the MeV regime. In particular, the photon energy for the energy distribution in figure 1b is the same as in figure 1a. In an alternative embodiment, the energy of the electron beam used in the imaging mode depicted in figure 1b is different to the energy used in the therapy mode depicted in figure 1a. In general, the electron beam has an energy sufficient for generating a photon beam with an energy in the range of therapy energies. This allows to use the same electron beam source since the energy of the electron beam emitted thereby cannot be adjusted over a wide range of energies comprising both, energies for generating therapy photon beams and energies for generating imaging photon beams with the same or a similar target.

However, the intensity of the electron beam 12 in figure 1b is less than the intensity of the electron beam in figure 1a as can be seen by the thickness of the arrows representing the electron beam 12. In contrast to the energy, electron beam sources, in particular LINACs can be easily adjusted to emit electron beams with substantially different intensities. Depending on the robustness of the imaging target, the electron beam source can as well be operated with same or similar intensities in both modes, the therapy mode (shown in figure 1a) and the imaging mode (shown in figure 1b). Using the same or similar intensities is not shown in figures 1a and 1b.

In the system shown in figure 1b, the target onto which the electron beam is emitted (with lower intensity than in figure 1a), is the imaging target 20b. In the imaging mode shown in figure 1b, the therapy target 20a is not arranged within the electron beam 12 but has been displaced from the electron beam 12 and has been replaced by the imaging target 20b. Imaging target 20b is shown in figure 1b in a symbolic representation and comprises at least one crystal. The crystals constituting the imaging target 20b are symbolically represented by diamonds in figure 1a and 1b. In figures 1a and 1b, the targets are presented only symbolically and in particular the thickness of the targets and other geometric properties are not reflected in the depiction.

The electron beam 12 (with lower intensity than in figure 1a) is emitted onto the target 20b, and, upon the interaction of the electron beam 12 with the target 20b, coherent bremsstrahlung 32 is generated and provides the imaging photon beam 32. Due to the distinct interaction principles between electron beam and imaging target 20b on one side and electron beam and therapy target 20a on the other side, the average energy of the imaging photon beam 32 is substantially lower than the energy of the therapy beam 30 shown in figure 1a. In particular, the amount of low energy photons (particularly suitable for imaging, e.g. < 100 keV or < 80 keV), i.e. the low energy part of the spectrum of imaging beam 32, is higher as compared to the prior art. Even though the imaging photon beam 32 and the therapy photon beam have similarities as regards their generation, origin and propagation properties (in particular the direction of propagation), the energy distribution of both photon beams substantially differs. In particular, due to the different energies, the photon beams are used for distinct purposes (imaging/therapy), which is represented by distinct reference signs.

The imaging photon beam 32 is directed towards the object 40, partly travels through the object 40 and is detected by the imaging detector 50. A part of the imaging beam 32 is absorbed within the object according to properties of the object 40, which can be provided by the intensity, photon number and/or photon energy distribution received by detector 50.

Target 20b comprises thin crystals, for example with a thickness between 5 and 50 µm and is consequently substantially thinner than the therapy target 20a. Even though the electron beam source 10 emits in imaging mode (depicted in figure 1b), an electron beam 12 with a lower intensity, a part of the electron beam passes the imaging target 20b due to the low thickness of the target 20b. This residual electron beam 34 is not desired and is directed away from the object 40. Thus, in a deflection area 70 a field is applied to the beams propagating from the target 20b, i.e. the residual electron beam 34 and the imaging photon beam 32. In the deflection area 70, for example a magnetic field is applied with the field direction being perpendicular to the propagation direction of the residual electron beam 34 (and of the imaging photon beam 32). The residual electron beam 34 is deflected within area 70 by the Lorentz force towards absorbing element 62, 62' (attracting and) absorbing the electrons as well as the resulting absorption bremsstrahlung. Electrode 62 attracts the residual electron beam 38 by having an appropriate potential, the residual electron beam 38 is absorbed (i.e. decelerated) within the electrode (e.g. a plate of copper or aluminium), and the absorption bremsstrahlung 37 resulting from the absorption of the residual electron beam 38 within the electrode 62 is absorbed in absorber 62', e.g. a plate of lead or any other shielding material. Electrode 62 and absorber 62' together form the absorbing element for absorbing the residual electron beam 38 and the absorption bremsstrahlung 37 generated thereby within the electrode 62. Absorption bremsstrahlung 37 is electromagnetic high energy irradiation critical to a patient.

Deflection radiation absorber element 60 absorbs microwave or IR irradiation 36, also denoted as deflection radiation, generated by the deflection of residual electron beam 38 and is provided by a metal sheet, e.g. a thin sheet of aluminium. Due to the low energy of the deflection radiation 36, the presence of deflection radiation absorber element 60 is not critical to the invention, in particular due to the fact that a casing (not shown) housing the inventive apparatus is suited to provide the deflection radiation absorber element 60.

Instead of deflecting the residual electron beam by a magnetic field, a static electric field can be applied, for example by applying a suitable potential below the potential of the residual electron beam to the blocking element 62. In both cases, the deflection is inherently linked with the generation of additional deflection radiation 36 (low energy) which is emitted in direction radial to path of propagation of the residual electron beam during reflection. The direction of propagation of the additional radiation 36 is opposite to the direction of the force applied to the residual electron beam 34 resulting from the field in the reflection area 70. In this way, the deflected residual electron beam 38 is directed towards the absorbing element 62 and away from object 40, and the deflection radiation 36 resulting from the deflection is directed towards the blocking element 60, opposed to blocking element 62 with regard to the imaging photon beam. Thus, also the deflection radiation 36 is directed away from the object 40. In figure 1b, the deflection is provided with a deflection angle of 45° which allows to direct the residual photon beam and the deflection radiation symmetrically away from the target 40. However, also other suitable geometries can be used as long as no part of the deflected residual electron beam 38, no part of the resulting deflection radiation 36 resulting from a deflection and no part of the absorption bremsstrahlung 37 (high energy) is emitted onto a point of the object area of object 40 irradiated by the imaging photon beam 32. In particular, the deflection is preferably provided in a way which ensures that neither the deflection radiation 36 nor the deflected residual electron beam 38 nor the bremsstrahlung 37 generated within the electrode 62 reaches the area, in which the object or in general the patient is located. Blocking element 60 can be any absorber or shielding suitable for absorbing or blocking the deflection radiation 36, and the absorbing element 62 can be any absorber, shielding, electrode or any combination thereof suitable for terminating the propagation of the deflected residual electron beam and suitable for absorbing the absorption bremsstrahlung 37.

In both figures 1a and 1b, the target is shown symbolically. In realizations of the inventive apparatus, an additional filter (in form of a plate of copper with a thickness of ca. 1 - 3 mm) is located downstream the target in order to absorb photons with very low energies. This filter is not shown in the filters for the sake of simplicity. Such a filter can be arranged at the location of reference sign 34. The filter absorbs photons with very low energies thereby reducing the dose (intensity) of undesired irradiation for a patient. Due to the very low energy (e.g. less than 30 keV or less than 60 keV) of the photons blocked by the filter, these photons are not suited to contribute to therapy or imaging. Rather, these photons are absorbed (due to their low energy) within surface areas of the patient and may harm the skin or a superficial area of the patient. Due to the low selectivity of the filter, also low energy imaging photons are absorbed. Since the invention allows to raise the amount of imaging photons within the spectrum of the imaging beam, the disadvantages resulting from the (mandatory) filter are at least partly compensated. Further, according to the invention, the blocking effects of the filter can be reduced in its, since the invention, in particular the imaging target, reduces the generation of harmful low energy photons. The imaging target can be used to specifically define the spectrum of generated photons, according to the properties of the filter for at least partly compensating negative influences of the filter on the generated spectrum.

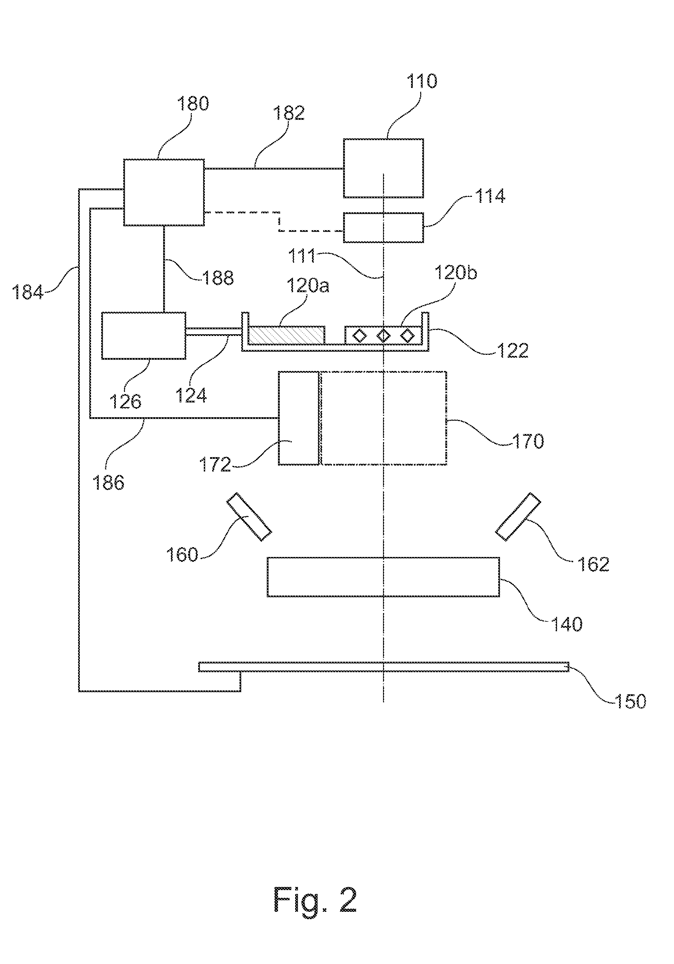

Figure 2 shows an embodiment of an apparatus according to the invention. In figure 2, three-digit reference signs are used beginning with "1". These reference signs are for referencing particular elements of figure 2 only, the reference signs itself do not refer or describe any technical features like crystal directions. In particular, the notation of the reference signs does not indicate any crystal direction. The apparatus shown in figure 2 comprises an electron beam source 110, electron beam optics 114, targets 120a, 120b attached onto a target holder 122. The target holder 122, and therewith the imaging target 120b and the therapy target 120a, is attached via a mechanical connection 124 to an actuator 126, which is adapted to move the targets in a direction perpendicular to the main propagation direction 111 of the electron beam source 110. The actuator 126 is attached to the holder 122 in order to arrange the imaging target 120b completely within the electron beam emitted by the electron beam source 110, or to arrange the therapy target 120a into the complete electron beam emitted by the electron beam source 110.

The apparatus of figure 2 further comprises a deflection device 172 which is adapted to generate a deflecting field within the deflecting area 170. The deflecting device can be provided by at least one coil having a longitudinal axis perpendicular to the main propagation direction 110 and being arranged in a distance to the main propagation direction 111. This offset guarantees that no part of the coil lies within the residual electron beam of imaging target 120b and the photon beams (therapy photon beam/imaging photon beam). The deflection device 172 is only symbolically represented in figure 2, the particular arrangement of the reflection device 172 in a physical implementation can substantially differ from the arrangement shown in figure 2. As an alternative to a magnetic deflection device using coils and deflecting the residual electron beam by a magnetic field, the deflection device 172 can be provided as electrostatic deflection device by an electrode or by an electrode pair or by an electrode, combined with an absorber located behind the electrode as shown by absorbing element 162. In the latter case, the arrangement differs from the depiction of figure 2, in which deflection device 172 and absorber 162 are distinct, individual components. In an example according to figure 2, an electrode can be arranged at the place of the symbolic representation of deflecting device 172, i.e. along the direction of propagation of the photon beams and the residual electron beam and perpendicularly offset to the main propagation direction 111.

The apparatus of figure 2 further comprises (blocking elements or) absorbing elements 160, 162. One of the absorbing elements 160, 162 is provided for absorbing the residual and deflected electron beam as well as the absorption bremsstrahlung generated thereby (e.g. the absorbing element 162), while the other of the absorbing elements 160, 162 (e.g. the deflection radiation absorber element 160) is provided for shielding, blocking or absorbing the additional bremsstrahlung, which is created by the deflection of the residual electron beam. It should be noted that the deflecting device 172, its deflection area and the absorber elements 160, 162 are arranged according to the properties within the deflection area 170, the properties of the residual electron beam and the relationships given by the definition of the Lorentz force and the electrostatic force according to the Maxwell equations.

The apparatus shown in figure 2 further comprises an object 140, i.e. an object area, in which a patient can be irradiated with the imaging photon beam and the therapy photon beam. In order to detect the intensity distribution of the therapy beam passing the object area 140, the apparatus comprises an image detector 150 extending along a plane perpendicular to the main propagation direction 111.

For control and evaluation purposes, the apparatus comprises a controller 180 and connections 182, 184, 186 and 188. Connection 182 connects the controller 180 to the electron beam source 110 and transmits control data to the electron beam source 110. The connection 182 connects the imaging detector 150 to the control 180 and transmits imaging data received by the detector 150 to the controller 180. Connection 186 connects the deflection device 172 to the controller 180 and transmits control signal from the controller 180 to the deflection device 172 for adjusting the deflection field. Connection 188 connects the controller 180 to the actuator 126 and transmits control information from the controller 180 to the actuator 126 to control the movement of target holder 122 and to control the operating mode of the device by appropriately arranging the selected target into the electron beam. The controller comprises a synchronisation unit or a synchronisation function for synchronising the intensity adjustments of electron beam source 110 with the movement and arrangement of the particular target by actuator 126. An optional connection between controller 180 and electron beam optics 114 transfers control data from the controller 180 to the electron beam optics 114, for example for appropriately defining the intensity distribution of the electron beam emitted by the electron beam source 110. In particular, the electron beam optics can be used for beam forming in order to appropriately adjust the intensity distribution to the desired radiation distribution at the object 140. Further, this optional connection shown with dashed lines allows to provide a scanning function for scanning a thin electron beam emitted by the electron beam source 110 onto the target. In particular in case of an imaging target comprising a plurality of crystals, the electron beam can be deflected to irradiate only the crystals and to neglect the space between the crystals. Further, if a combined target is used, the scanning function provided by the electron beam optics 114 and the control 180 allows to select the mode of operation. The mode of operation can be selected by scanning only the crystals within the combined target in imaging mode and to scan only the residual therapy target sections of the combined target in therapy mode. Further, the electron beam optics can be provided for broadening the electron beam of electron beam source 110 during the imaging mode for providing a broad field of view and to adapt the intensity distribution of the electron beam during therapy mode, for example by focussing and/or scanning the electron beam only on the directions linked with a radiation of a particular object area. Thus, when emitting onto the imaging target, the electron beam source (together with the electron beam optics 114) provides a broadened beam with a divergence higher than the therapy target by the electron beam source. If using a particular intensity distribution during therapy mode (i.e. during emitting the electron beam onto the therapy target), the control 180 can be used for overlaying the imaging data received from the detector 150 with an image of the intensity distribution of the therapy photon beam directed towards the object during therapy mode.

Control 180 can be provided by a programmable microprocessor or controller, in combination with software or software pieces implementing some or all of the above mentioned functions. The connections 182 to 188 can be provided by known data link connections, for example wired data link connection in serial transmission mode.

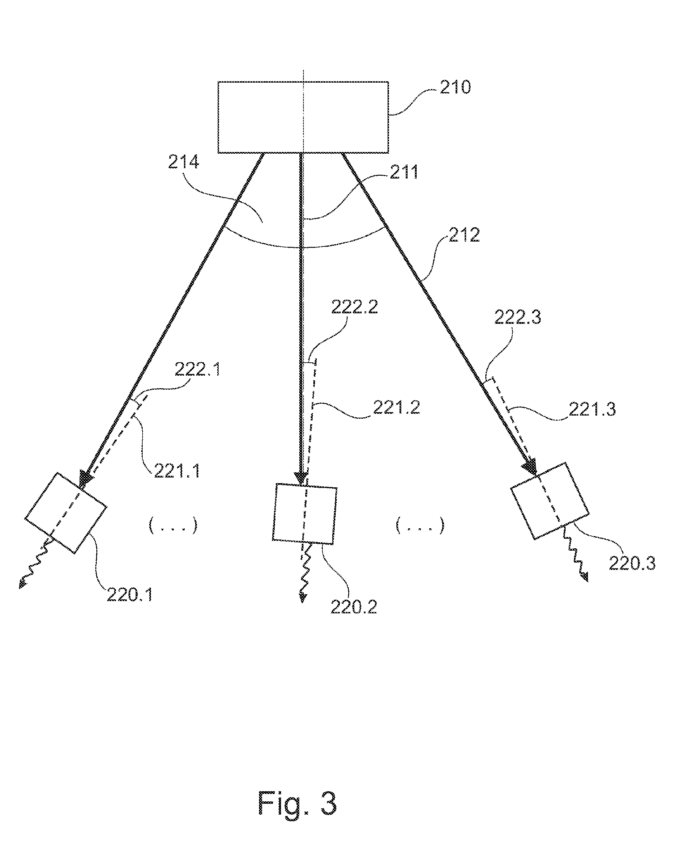

Figure 3 shows a preferred embodiment of an imaging target to be used with the invention. Figure 3 shows symbolically an electron beam source 210 (optionally combined with electron beam optics), the electron beam source 210 emitting an electron beam 212 with a certain angle of divergence 214 along a main propagation direction 211. Figure 3 shows an arrangement according to the invention during imaging mode, i.e. during the electron beam 212 is emitted onto the imaging target. The emitting target comprises a plurality of crystals 220.1, 220.2 and 220.3. Each of the crystals has a lattice with a primary direction 221.1-221.3 shown with dashed lines. Between the respective primary directions of the lattice 221.1 and the corresponding propagation direction along which the particular electron beam is emitted onto the respective crystal a certain angle 222.1-222.3 is given. The primary access 221 of all crystals is tilted to the propagation direction of the electron beam section, which is emitted on the respective crystal 220.1-220.3 by substantially the same angle 222.1-222.3. Thus, all crystals are tilted by the same angle to the electron beam. Since the electron beam 212, 214, the outer crystals are tilted by an additional angle given by the inclination between electron beam section emitted onto the outer crystal and main propagation direction 211. Consequently, the inner crystal which lies in the main propagation direction 211 is only tilted by the desired angle 222.2, without additional inclinations due to diverging propagation directions of the electron beam section emitted on the crystal. Angle 221.1-221.3 is preferably within a range of 1° +/- 0,8°. In figure 3, this angle has been increased for illustrative purposes. In addition, all crystals 220.1-220.3 are preferably attached to each other, for example by attaching all crystals to a supply or substrate (not shown). The total tilting angle of the crystals 220 increasing with the distance to the main propagation direction 221 can be provided by attaching all crystals onto a substrate with the same tilting angle (which simplifies the manufacturing) and by bending the substrate after the crystals have been attached thereto. The bending angle and the curvature can be adapted to the divergence of the electron beam. In this way, all primary directions of the crystals have approximately the same inclination to the electron beam section which is emitted thereon. The divergence of the electron beam can be provided by scanning the electron beam within a predefined solid angle thereby distributing the intensity of the electron beam homogenously over the cross section of the target. If a plurality of crystals is used as depicted in figure 1, the crystals comprise at least one inner crystal (for example crystal 220.2) onto which an inner section of the beam is emitted onto as well as at least one outer crystal (for example 220.1 and 220.3) onto which an outer section of the beam is emitted onto. Due to the divergence, an outer beam section is inclined to the main propagation direction. The at least one outer crystal is more inclined towards the main direction of propagation of the electron beam than the at least one inner crystal. In case of a diverging electron beam as depicted in figure 3, the electron beam comprises inclined beam sections with directions of propagation different to the main direction of propagation. The imaging target comprises at least two crystals as shown in figure 3, wherein the two crystals have distinct lattice orientations.

According to another aspect of the invention, the electron beam is diverging and is emitted onto an array of crystals. Each crystal has a radiated surface on which a respective beam section is emitted onto. The radiated surfaces are inclined to a center line of propagation, i.e. inclined to the main direction of propagation of the electron beam by a radial angle of inclination changing (preferably increasing) with the distance between the particular radiated surface of the radiated surfaces of the crystals and the main direction of propagation. The inclination between electron beam and crystal changing with the distance compensates the inclination of non-central electron beam sections to the main propagation direction of the electron beam. Thus, the incidence angle of all electron beam sections onto their respective crystal is substantially independent from the inclination of the electron beam section to the main propagation direction of the electron beam. This increase or change can be a linear increase according to the distance or can be proportional to the square of the distance. In this way, the crystals are tilted by an angle increasing with the distance to the center which at least partly compensates the inclination of beam sections diverging to the main propagation direction such that each crystal is inclined to the particular electron beam section emitted thereon with approximately the same angle. Preferably, the crystals providing the imaging targets are arranged in a laminar arrangement, for example in form of a plain or a convex curvature, preferably rotation-symmetric to the main propagation of the electron beam. The crystals are arranged side by side, wherein (periodical) spacings between the crystals are provided. Any electron beam section propagating through these spacings can be removed by deflecting the residual electron beam using a deflection devise as described above. Alternatively, the electron beam source and its optics can be provided to scan an electron beam onto the crystals, wherein the spacings are excluded.

According to simulation results based on a model representing the invention, an electron beam with an energy of 6 MeV, which is suited for generating therapy radiation using a conventional therapy target generates a bremsstrahlung spectrum when emitted onto a thin diamond crystal (between 5 and 50 µm) with more than 25 % of spectrum energy below 80 keV with an average energy of about 250 keV. At the same time, less than 10 % of the photons have an energy of less than 40 keV. In this regard, such low energy photons are not suited for imaging but have to be blocked by a filter between target and object (preferably arranged in close distance and directly after the target). As already discussed above, a mandatory filter is used in the prior art to filter out undesired low energy photons, which do not support the imaging but only increase the radiation dose of the patient. Such low energy photons (less than 40 keV) are absorbed in the skin or in a superficial area of the patient. The energy of such photons in not sufficient to at least partly propagate through the complete human body. Since the invention allows to specify the spectrum of the imaging beam with reduced undesired slow energy components (e.g. less than 40 keV) and to increase the spectrum components suitable for imaging (e.g. more than 40 keV and less than 100 keV), the mandatory filter can be provided with less restrictive blocking properties. This (and in particular the reduced undesired slow energy components) allows a higher efficiency and the specific focus onto photon energies suitable for imaging.

In the context of the simulation results, the tilt angle between primary lattice direction and beam direction of the electron beam impinging thereon was between 0.6° and 2°. Even if a higher energy of 18 MeV is used for the electron beam, more than 30 % of the spectrum energy was found to be below 100 keV for a tilt angle between 0.2° and 1°, and even below 70 keV for a tilt angle between 0.1 and 0.4°.

### Reference Signs

- 10, 110,210: electron beam source
- 12,212: electron beam
- 20a, 120a: therapy target
- 20b, 120b, 220.1- 220.3: imaging target
- 30: therapy photon beam
- 32: imaging photon beam
- 34: residual electron beam
- 36: additional bremsstrahlung
- 37: deflection radiation
- 38: deflected residual electron beam
- 40, 140: object, object area
- 50, 150,: imaging detector
- 62, 62', 162: absorbing element for residual electron beam / bremsstrahlung resulting therefrom (62: electrode, 62': absorber)
- 60, 160: absorbing elements for deflection bremsstrahlung
- 70, 170: deflection area
- 111, 121: main direction of propagation
- 114: electron beam optics
- 122: target holder
- 124: mechanical connection between actuator and target holder
- 126: actuator
- 172: deflecting device
- 180: controller
- 182-188: data/control connection
- 214: angle of divergence
- 221.1-221.3: crystal lattice direction
- 222.1-222.3: inclination angle between crystal lattice and electron beam

## Claims

1. Method for image guided radiotherapy, comprising the steps:
an electron beam source (10, 110, 210) emitting an electron beam (12, 212) onto an imaging target (20b, 120 b, 220.1-220.3), the electron beam (12, 212) having an energy suitable for generating a therapy photon beam (30), **characterized by** the step of: emitting the electron beam (12, 212) onto at least one surface area of at least one crystal providing the imaging target (20b, 120 b, 220.1-220.3), wherein the electron beam (12, 212) is emitted onto the crystal in an angle to a crystal axis of the crystal's lattice, the crystal providing coherent bremsstrahlung upon interaction with the electron beam as an imaging photon beam (32) with an energy lower than the energy of the therapy photon beam (30); and directing the imaging photon beam (32) towards an object.

2. Method of claim 1, further comprising the step of: generating the therapy photon beam (30) by emitting the electron beam (12, 212) onto a therapy target, wherein the electron beam (12, 212) is emitted onto the therapy target and onto the imaging target (20b, 120 b, 220.1-220.3) alternatingly, subsequently or concurrently, and the therapy target (20a, 120a) provides the therapy photon beam (30) upon interaction of the therapy target (20a, 120a) with the electron beam (12, 212); and the step of directing the therapy photon beam (30) towards the object (40, 140).

3. Method of claim 2, further comprising the step of: arranging the imaging target (20b, 120 b, 220.1-220.3) between the electron beam source (10, 110, 210) and the object (40, 140), when the imaging photon beam (32) is provided, and arranging the therapy target (20a, 120a) between the electron beam source (10, 110, 210) and the object (40, 140), when the therapy photon beam (30) is provided, and wherein the electron beam (12, 212) is emitted onto the therapy target (20a, 120a) and onto the imaging target (20b, 120 b, 220.1-220.3) alternatingly or subsequently, and the imaging target (20b, 120 b, 220.1-220.3) is replaced by the therapy target (20a, 120a) and vice versa by translational or rotational movement of a target holder (122) on which the imaging target (20b, 120 b, 220.1-220.3) and the therapy target (20a, 120a) are arranged.

4. Method of one of the preceding claims, wherein the electron beam (12, 212) is emitted as a divergent beam or is scanned over a given solid angle and emitted onto the surface area of one or a plurality or an array of crystals, the plurality of crystals being arranged in a plane substantially perpendicular to the electron beam (12, 212).

5. Method of one of the preceding claims, wherein the angle between the electron beam (12, 212) and the crystal axis is at least 0.05°, at least 0.1°, at least 0.15° or at least 0.2°, and being not greater than 60°, not greater than 50°, not greater than 45°, not greater than 30°, not greater than 20°, not greater than 15°, greater than 10°, not greater than 5°, not greater than 2° or not greater than 1.8°, and the at least one surface area extends in a plane perpendicular to the crystal axis or to another axis of the crystal, or wherein the angle is an acute angle.

6. Method of one of the preceding claims, wherein the electron beam (12, 212) is emitted onto at least one crystal of elemental solid matter or onto at least one quasicrystal providing the crystal, wherein at least one crystal of elemental solid matter providing the imaging target (20b, 120 b, 220.1-220.3) is preferably made of Be, B, Diamond, Graphite, Si, Mg, Se, Ti, Cr, Fe, Ni, Cu, Ge, Z, Yr, Nb, Mo, Pd, Ag, Ho, Ta, W, Au, Tb or Be₂C, and the at least one quasicrystal providing the imaging target (20b, 120 b, 220.1-220.3) is preferably made of aluminium alloys, Al-Li-Cu, Al-Mn-Si, Al-Ni-Co, Al-Pd-Mn, Al-Cu-Fe, Al-Cu-V or of Cd-Yb, Ti-Zr-Ni, Zn-Mg-Ho, Zn-Mg-Sc, In-Ag-Yb, or Pd-U-Si.

7. Method of one of the preceding claims, wherein the electron beam is emitted onto the at least one crystal providing the imaging target (20b, 120 b, 220.1-220.3), wherein the at least one crystal is provided with a thickness along the direction of propagation of the electron beam (12, 212) of not more than 1 mm, 0.5 mm, 200 µm, 100 µm, 50 µm or 20 µm and at least 500 nm, 1 µm, 2 µm, 5 µm or 100 µm or in a range of 5 µm and 50 µm.

8. Method of one of the preceding claims, further comprising: directing any residual electron beam remaining after the interaction between the at least one crystal and the electron beam (12, 212) emitted thereon away from the object (40, 140) by applying an electrostatic or magnetic field or a combination thereof to the residual electron beam (34), by absorbing the residual electron beam in an absorbing element (62, 62') and by absorbing substantially all photons of an absorption bremsstrahlung (37) generated by the step of absorbing the residual electron beam, wherein substantially all photons generated by the application of the field as deflection radiation (36) are blocked or produced to propagate in a direction away from the object (40, 140).

9. Method of one of the preceding claims, further comprising: cooling the at least one crystal by a heat sink, preferably by a heat sink based on heat transfer to a medium, the medium comprising air of the environment, water, oil, fluidic nitrogen, fluidic helium, carbon dioxide.

10. Apparatus for image guided radiotherapy, comprising: an electron beam source (10, 110, 210) and an imaging target (20b, 120 b, 220.1-220.3), the electron beam source (10, 110, 210) being oriented towards the imaging target (20b, 120 b, 220.1-220.3) **characterized in that** the electron beam source (10, 110, 210) is adapted to provide an electron beam (12, 212) propagating along a direction towards the imaging target (20b, 120 b, 220.1-220.3) with an energy suitable for generating a therapy photon beam (30), wherein the imaging target (20b, 120 b, 220.1-220.3) is provided by at least one crystal having at least one surface area oriented towards the electron beam source (10, 110, 210), the electron beam source (10, 110, 210) and the at least one crystal are arranged to each other with the direction, in which the electron beam source (10, 110, 210) emits the electron beam being in an acute angle to a crystal axis of the crystal's lattice, the crystal thereby being adapted to provide coherent bremsstrahlung upon interaction with the electron beam (12, 212) as an imaging photon beam (32) with an energy lower than the energy of the therapy photon beam (30), wherein an object area (140) is located at a side of the imaging target (20b, 120 b, 220.1-220.3) opposed to the electron beam source (10, 110, 210).

11. Apparatus of claim 10, further comprising: a therapy target (20a, 120a) and a target holder (122), the therapy target (20a, 120a) as well as the imaging target (20b, 120 b, 220.1-220.3) being affixed to the target holder (122), wherein the target holder (122) is movably supported relative to the electron beam source (10, 110, 210) for providing a translational or rotational degree of freedom with regard to the electron beam source (10, 110, 210), the apparatus further comprising an actuator (126) connected to the target holder (122) as well as a controller (180) connected thereto, the controller (180) being adapted and connected to control the actuator to selectively exchange the imaging target (20b, 120 b, 220.1-220.3) and the therapy target (20a, 120a) alternatingly, subsequently or concurrently, and to accordingly place the respective target into the direction of propagation of the electron beam (12, 212).

12. Apparatus of claim 10 or 11, wherein the electron beam source (10, 110, 210) is adapted to emit the electron beam (12, 212) as a divergent beam, or wherein the apparatus further comprises a electron beam deflector (114) having a magnetic or electrostatic field generator adapted to provide a changing magnetic or electrostatic field deflecting the electron beam emitted by the electron source and adapted to scan the electron beam over a given solid angle, the at least crystal being by one, a plurality or an array of crystals, each having a surface area oriented towards the electron beam source (10, 110, 210), wherein, in case of the plurality or an array of crystals, the crystals are arranged in a plane perpendicular to the direction of the electron beam.

13. Apparatus of one of claims 10 - 12, the at least one crystal is of at least one chemical element or is a quasicrystal.

14. Apparatus of one of claims 10 - 12, further comprising a deflector device (172) arranged between the object area (140) and the imaging target (20b, 120 b, 220.1-220.3), the deflector device (172) comprising an electrostatic field generator and/or an magnetic field generator for deflecting any residual electron beam (34) remaining from the electron beam after interaction with the imaging target (20b, 120 b, 220.1-220.3) away from the object area (140), the apparatus further comprising an absorbing element (62, 62') suitably arranged relative to the deflected electron beam (38) and to the deflector device (172) to receive substantially the complete residual electron beam (34), the absorbing element comprising an electrode (62) receiving the residual electron beam (34), and an absorber (62') located behind the electrode (62) arranged relative to the electrode (62) suitable to receive substantially all absorption bremsstrahlung (37) generated by the deflected residual electron beam (38) within the electrode; and a deflection radiation absorber element (62, 162) being arranged and adapted to absorb deflection radiation produced by the interaction between the deflector device (172) and residual electron beam (34), wherein the deflector device (172) is arranged and adapted to direct the residual electron beam (38) and the deflection radiation (36) away from the object area.

15. Use of an electron beam source (10, 110, 210) as a common source for generating both, an imaging photon beam (32) as well as a therapy photon beam (30) with a photon energy higher than the photon energy of the imaging photon beam (32) by using a therapy target (20a, 120a) and an imaging target (20b, 120 b, 220.1-220.3) comprising at least one crystal, wherein the therapy photon beam (30) is generated by irradiating the therapy target with the electron beam source (10, 110, 210) and the imaging photon beam (32) is generated by arranging the imaging target (20b, 120 b, 220.1-220.3) relative to the electron beam source (10, 110, 210) with an angle between a crystal axis of the crystal's lattice and a direction of propagation of the electron beam source (10, 110, 210), thereby generating the imaging photon beam (32) as coherent bremsstrahlung resulting from the interaction between the electron beam source's beam and the imaging target (20b, 120 b, 220.1-220.3).
